(19) Europäisches Patentamt
European Patent Office
Office européen des brevets

(11) Publication number: 0 035 413
B1

(12) # EUROPEAN PATENT SPECIFICATION

(45) Date of publication of patent specification: 05.09.84

(51) Int. Cl.$^3$: **C 07 D 501/36,** C 07 D 501/46, A 61 K 31/545

(21) Application number: 81300917.2

(22) Date of filing: 05.03.81

(54) **Antimicrobial 7-(alpha-acylamino-alpha-arylacetamido)-3-(substituted methyl)cephalosporin compounds, their compositions and production.**

(30) Priority: 05.03.80 JP 28536/80
05.03.80 JP 28537/80
05.03.80 JP 28538/80

(43) Date of publication of application:
09.09.81 Bulletin 81/36

(45) Publication of the grant of the patent:
05.09.84 Bulletin 84/36

(84) Designated Contracting States:
BE CH DE FR GB IT LI

(56) References cited:
US-A-3 954 734
US-A-4 068 074
US-A-4 160 087
US-A-4 165 373

CHEMICAL ABSTRACTS, vol. 92, no. 13, March 31, 1990, page 667, abstract 111042j Columbus, Ohio, USA

The file contains technical information submitted after the application was filed and not included in this specification

(73) Proprietor: SUMITOMO CHEMICAL COMPANY, LIMITED
15 Kitahama 5-chome Higashi-ku
Osaka-shi Osaka-fu (JP)

(72) Inventor: Yamada, Hirotada
No. 1-808, Takahata-cho No. 1
Nishinomiya-shi Hyogo (JP)
Inventor: Jimpo, Kiyokazu
No. 273, Kinda-cho 1-chome Moriguchi-shi
Osaka (JP)
Inventor: Okuda, Takao
No. 10-3-337, Sonehigashi-machi 2-chome
Toyonaka-shi Osaka (JP)
Inventor: Noguchi, Hiroshi
Apt. 406, No. 15-10 Kusunoki-cho
Ashiya-shi Hyogo (JP)
Inventor: Tobiki, Hisao
No. 11-401, Arise No. 1157 Ikawadani-cho
Tarumi-ku Kobe-shi Hyogo (JP)

(74) Representative: Diamond, Bryan Clive
Gee & Co. Chancery House Chancery Lane
London WC2A 1QU (GB)

EP 0 035 413 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to cephalosporin compounds. More particularly, it pertains to antimicrobial 7-(α-acylamino-α-arylacetamido)-3-(substituted methyl)cephalosporin compounds, and their composition and use, and to a process for producing the same.

The cephalosposporanic acid nucleus is

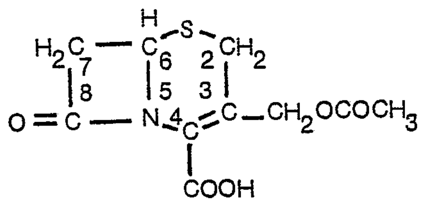

Antibiotic derivatives thereof are known including one where the 3-position bears a

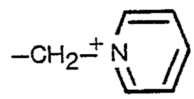 group (Cephaloridin),

and where the 7-position bears various substituents such as

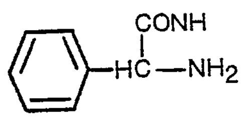 (Celafalexin, Celfaloglycin).

However, the known cephalosporins are not all effective against a wide range of bacteria.

In our U.S. Specification No. 4,160,087, we disclose cephalosporin derivatives wherein there is a 7-substituent:

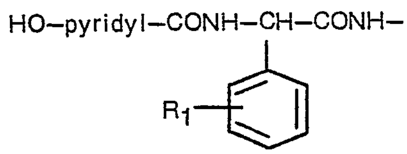

$R^1 = —NH_2$, —OH, $—NHCONH_2$ or $—CH_2OH$, and a 3-substituent which can be:

$$—CH_2—S—Het$$

wherein Het is a 5- or 6-membered heterocyclic group containing 1 to 4 N, O and S atoms.

We have now found new cephalosporin derivatives having preferred combinations of substituents which give superior pharmacological results.

According to the present invention, a 3,7-substituted cephalosporin compound is of the general formula:

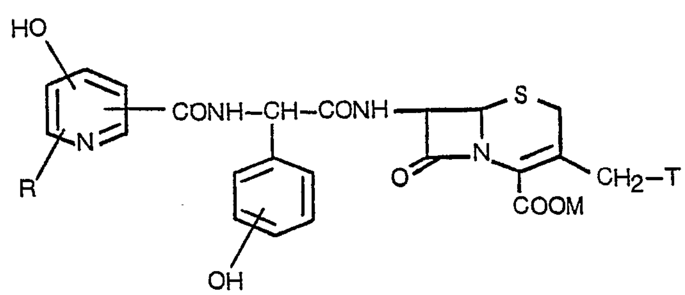 (I)

wherein R represents an alkyl group of up to 4 carbon atoms; T represents a group of one of the formulae:

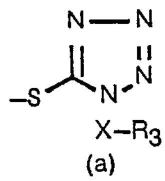

(a)

or

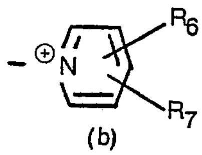

(b)

wherein $R_3$ represents a carboxyl or sulfo group, X represents an alkylene group of up to 4 carbon atoms, and $R_6$ and $R_7$ each represents a hydrogen atom, a hydroxy group, a carbamoyl group, a carboxy group or an alkyl group of up to 4 carbon atoms; M is a hydrogen atom, a pharmaceutically acceptable non-toxic cation, or when T is a group of the aforesaid formula (b), M may be an anionic charge.

Preferably in a tetrazole group T of formula (a), the alkylene group X is a lower alkylene group of up to 4 carbon atoms and a pyridyl group T of formula (b) has a single $R_6$ or $R_7$ substituent which is not a lower alkyl group. The nature of the $R^3$, $R^6$ or $R^7$ substituents can have an important effect on the properties of the compound.

The present invention also provides antimicrobial compositions comprising an antimicrobially effective amount of the cephalosporin compound of the formula (I) or its pharmaceutically acceptable non-toxic salt and a carrier or diluent. It further provides a process for producing the cephalosporin compounds of the formula (I).

The compounds of the present invention represented by the formula (I) and non-toxic salts thereof exhibit a strong antimicrobial activity against gram-positive and gram-negative bacteria including various microorganisms to which many of the commercially available cephalosporin type antibiotics are not effective, for example, *Pseudomonas aeruginosa,* indole-positive *Proteus, Serratia* or *Enterobacter aerogenes.*

Further, the compounds of the invention and non-toxic pharmaceutically acceptable salts thereof have good water-solubility and excellent pharmacokinetic properties, such as their increased and prolonged serum level in a living body. Thus, they are useful as antimicrobial agents for preventing and treating various bacterial infections caused by said microorganisms in humans and animals, including poultry and domestic animals, without causing any serious side-effects.

For the treatment or prevention of such infectious diseases, the compounds of this invention, either individually or in combination with a pharmaceutically acceptable carrier or diluent, or one or more other active ingredient, e.g. chemotherapeutic agent, can be administered parenterally to a subject.

The dosage of the compounds of the formula (I) of this invention will vary the body weight, age and condition of an individual subject, the kind of bacteria, and the pharmacokinetic properties of the particular compound chosen. Although the particular dosage will be determined by a physician taking these factors into consideration, the compounds of the formula (I) are, in general, most desirably administered parenterally at a doasge of from 5 mg/kg of body weight/day to 150 mg/kg of body weight/day, preferably from 15 mg/kg of body weight/day to 100 mg/kg of body weight/day in a single dose or in multiple doses 2 to 5 times daily.

The compound of the invention can comprise from 0.1 to 99% by weight of any pharmaceutical composition.

The compounds of this invention can also be administered orally in compositions such as tablets, capsules or granules, e.g. in admixture with conventional pharmaceutical excipients such as calcium carbonate, lactose, talc, starch, gelatin or magnesium stearate; other compositions can be in the form of suppositories or ointments.

For parenteral administration the compounds of this invention may be used in the form of a sterile solution or suspension containing additionally a pharmaceutically acceptable diluent or carrier such as water, saline solution, Ringer's solution, glycerin or polyethylene glycol. These preparations or formulations may also contain suitable auxiliary materials, such as stabilizers, buffer substances, wetting agents, emulsifiers, local anesthetics or salts which regulate the osmotic pressure.

The $\alpha$-carbon atom in the side chain at the 7-position in the formula (I), i.e. a phenylglycine moiety, is an asymmetric center and each compound of this invention can exist in two optical isomers. The present invention includes in its scope these two isomers (D-diastereomer and L-diastereomer) as well as a DL-form, with a preferred compound being a D-form.

Examples of said pharmaceutically acceptable non-toxic cation of the compounds of the formula (I) are a sodium salt, a potassium salt, a calcium salt, a magnesium salt, a triethylamine salt, a diethanolamine salt, a morpholine salt, a procaine salt, an L-arginine salt and an L-lysine salt.

With respect to the cephalosporin compounds (I) wherein T represents said pyridinium group of the formula:

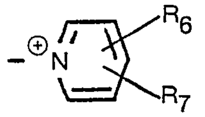

(b)

wherein $R_6$ and $R_7$ are as defined above, M may be a hydrogen atom or an anionic charge forming an inner salt or betaine with the pyridinium group as defined above. In the former case, the cephalosporin compounds are in the form of the pyridinium salt such as pyridinium chloride. In the latter case, they may be represented by the formula:

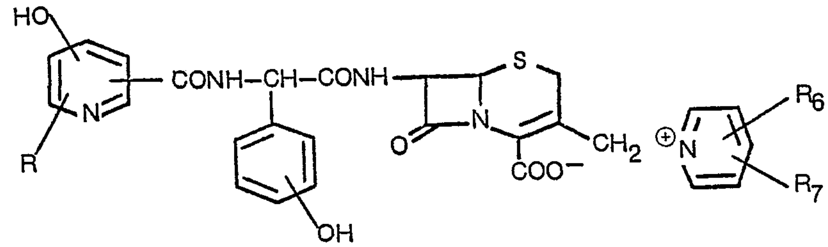

wherein R, $R_6$ and $R_7$ are as defined above.

The cephalosporin compounds of the present invention and the salts thereof can be prepared by reacting a carboxylic acid of the formula (II)

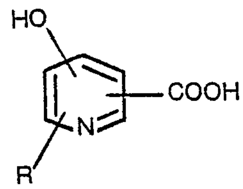

(II)

or a reactive derivative thereof, with a compound of the formula (III)

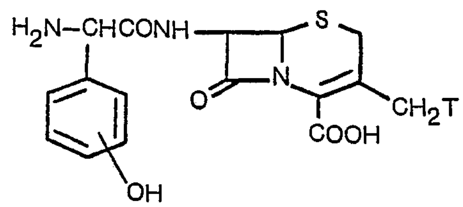

wherein T is as defined above, or a salt or a derivative of the compound of the formula (III).

More particularly, the reaction between the compound of the formula (II) or a salt or a reactive derivative thereof and the compound of the formula (III) or a salt or a derivative thereof is generally conducted in an inert solvent. Examples of the inert solvents are polar solvents such as dichloromethane, chloroform, acetone, tetrahydrofuran, dioxane, acetonitrile, methyl isobutyl ketone, ethyl alcohol, dimethylformamide, dimethyl sulfoxide, hexamethylphosphoric triamine or sulforane, non-polar solvents such as benzene, toluene, petroleum ether or *n*-hexane, or a mixture thereof.

The term "reactive derivative of the carboxylic acid represented by the formula (II)" means a reactive derivative of the carboxyl group, for example, an acid halide, an acid anhydride, an acid azolide, an active ester or an acid azide. Examples of the reactive derivatives are mixed acid anhydrides with an acid such as a dialkylphosphoric acid, phenylphosphoric acid, diphenylphosphoric acid, dibenzylphosphoric acid, dialkylphosphorous acid, methanesulfonic acid, toluenesulfonic acid, naphthalenesulfonic acid, alkylcarbonic acid, aliphatic carboxylic acid (e.g. pivalic acid, pentanoic acid, isopentanoic acid, 2-ethyl-butanoic acid), aromatic carboxylic acid or symmetric acid anhydride; acid azolides with imidazole, a substituted imidazole, dimethylpyrazole, triazole or tetrazole; or active esters such as cyanomethyl ester, methoxymethyl ester, *p*-nitrophenyl ester, 2,4-dinitrophenyl ester, trichlorophenyl ester, pentachlorophenyl ester, methanesulfonylphenyl ester, phenylthiophenyl ester, *p*-nitrophenylthio ester, *p*-cresylthio ester, carboxymethylthio ester, pyranyl ester, pyridyl ester, piperidyl ester or 8-quinolylthio ester; or active esters with N,N'-dimethylhydroxylamine, 1-hydroxy-2(1H)-pyridone, N-hydroxysuccinimide or N-hydroxyphthalimide.

When the carboxylic acid of the formula (II) is used in the form of a free carboxylic acid or a salt thereof, the reaction is preferably conducted in the presence of a condensing agent, for example, N,N'-dicyclohexylcarbodiimide, N-cyclohexyl-N-morpholinoethylcarbodiimide, N-cyclohexyl-N-(4-diethylaminocyclohexyl)-carbodiimide, N,N'-diethylcarbodiimide, N,N'-diisopropylcarbodiimide, N-ethyl-N-(3-dimethylaminopropyl)carbodiimide, N,N'-carbonyldi-(2-methylimidazole), pentamethyleneketene-N-cyclohexylimine, diphenylketene-N-cyclohexylimine, alkoxyacetylene, 1-alkoxy-1-chloroethylene, trialkyl phosphite, ethyl polyphosphate, isopropyl polyphosphate, phosphorus oxychloride, phosphorus trichloride, thionyl chloride, oxalyl chloride, triphenylphosphine, 2-ethyl-7-hydroxybenzisoxazolium salt, 2-ethyl-5-(*m*-sulfophenyl)isoxazolium hydroxide inner salt or (chloromethylene)dimethylammonium chloride.

In this manner, any amidation agents which have generally been used in the fields of peptide chemistry or penicillin and cephalosporin chemistry can be used in preparing the compounds of the formula (I) in the present invention.

A salt of a compound of the formula (III) can be an alkali metal or alkaline earth metal salt such as the sodium, potassium or calcium salt, organic base salt such as the trimethylamine, triethylamine, quinoline or collidine salt, organic sulfonic acid salt such as toluenesulfonic acid, naphthalenesulfonic acid or tetralinsulfonic acid salt; and a derivative of a compound of the formula (III) can be a carboxyl derivative such as one having protected carboxyl groups such as in the form of esterified or amidated carboxyl groups or in the form of acid anhydrides.

The protected carboxyl derivatives are those having a group which is capable of easily providing a carboxylic acid group by cleavage after acylation and preferably those providing a free carboxylic acid by, for example, solvolysis, e.g. hydrolysis or alcoholysis in an acidic or weakly alkaline medium, hydrogenolysis, reduction, oxidation, nucleophilic substitution, photoreaction or enzymatic reaction.

The above-described protected carboxyl derivatives are those having a protective group for carboxylic acid generally used in the field of peptide chemistry or in the field of penicillin and cephalosporin chemistry such as a silyl ester, an organo-tin ester, a toluenesulfonylethyl ester, a *p*-nitrobenzyl ester, a benzyl ester, a phenacyl ester, a 2-furylmethyl ester, a diphenylmethyl ester, a substituted diphenylmethyl ester, *p*-methoxybenzyl ester, a trityl ester, a benzoyloxymethyl ester, a lower alkanoyloxymethyl ester, a dimethylmethyleneamino ester, a *p*-nitrophenyl ester, a methylsulfonylphenyl ester, a methylthiophenyl ester, a *t*-butyl ester, a 4-picolyl ester, an iodoethyl ester, a trichloroethyl ester, a phthalimidomethyl ester, a 3,4-dimethoxy or 3,5-dimethoxybenzyl ester, a 2-nitrobenzyl ester, a 2,2′-dinitrobenzyl ester, an acetyloxycarbonyl group, a trichloroethyl ester, a

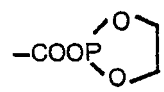

group, a —CONH=CHR′ group wherein R′ is an alkyl or aryl group, or a

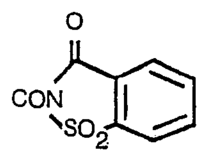

group.

The above-described derivatives of the compounds of the formula (III) can be used in the form of salts thereof such as the hydrochloride, toluenesulfonate, naphthalenesulfonate or tetralinsulfonate.

In the case where the above derivative used is in the form of a silyl ester, the derivative may have another silyl group on other moieties which can be silylated, i.e. hydroxy groups or amino groups.

The temperature of the reaction between the carboxylic acid represented by the formula (II) or a reactive derivative thereof and the 7-amino-acylamido-cephalosporin represented by the formula (III) or a salt or derivative thereof is not limited, but is generally lower than 50°C.

Alternatively, the compounds represented by the formula (I) can be prepared by a known process comprising reacting a $\alpha$-acylamino-$\alpha$-arylacetamidecephalosporin represented by the formula (IV)

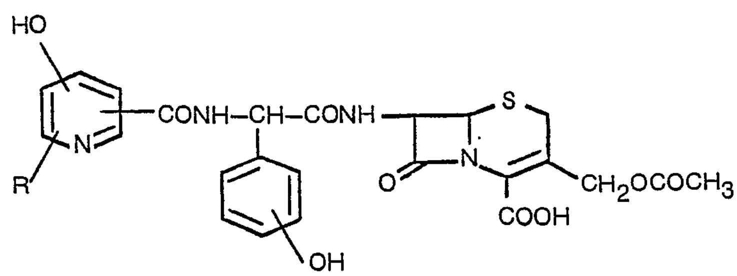

(IV)

wherein R is as defined above with a compound of one of the formulae

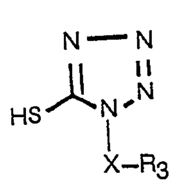

(Va)

or

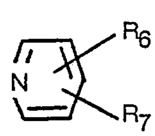

(Vb)

wherein $R_3$, $R_6$, $R_7$ and X are as defined above, or a salt thereof. Such a known process is disclosed in Japanese Patent Publications Nos. 12136/71, 2340/71 and 14734/71, Japanese Patent Application (OPI) No. 68593/73 (the term "OPI" as used herein refers to a "published unexamined Japanese patent application"), Journal of Chemical Society, *1965*, p. 5015, Belgian Patent 864—459, Journal of Medicinal Chemistry, Vol. 17, p. 1312 (1974) and Journal of Organic Chemistry, Vol. 32, p. 500 (1967).

Further, the compounds represented by the formula (I) can also be prepared by reacting the acylaminocarboxylic acid represented by the formula (VI)

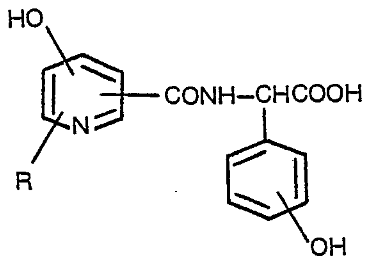

(VI)

wherein R is as defined above, or a reactive derivative thereof, with a compound represented by the formula (VII)

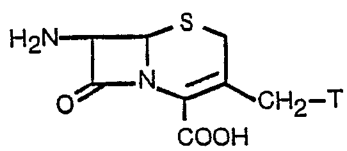

(VII)

wherein T is as defined above, or a derivative thereof.

The reaction between the acylaminocarboxylic acid of the formula (VI) or a derivative thereof and the compound of the formula (VII) can be conducted in the same manner as described previously for the reaction between the compound (II) and the compound (III).

The present invention is further illustrated by the following illustrative Examples.

Example 1

Preparation of 7-[D-$\alpha$-(4-hydroxy-6-methylpyridine-3-carboxyamido)-$\alpha$-(4-hydroxyphenyl)-acetamido]-3-(1-carboxymethyl-1H-tetrazol-5-ylthiomethyl)-3-cephem-4-carboxylic acid

8.8 g of a triethylamine salt of 7-[D-$\alpha$-(4-hydroxy-6-methylpyridine-3-carboxyamido)-$\alpha$-(4-hydroxyphenyl)acetamido]-cephalosporanic acid, 2.36 g of sodium bicarbonate and 3.0 g of 1-carboxymethyl-5-mercapto-1H-tetrazole were added to 117 ml of a phosphate buffer solution (pH 6.0), and the resulting mixture was allowed to react at 50°C for 16 hours and then at 60°C for 3 hours with stirring. The reaction mixture was allowed to cool to room temperature and adjusted to pH 2.0 with 6N hydrochloric acid. The precipitated crystals were collected by filtration and dried over phosphorus pentoxide under reduced pressure to give 6.35 g of a crude product of the compound named at the head of this Example.

The crude product thus-obtained was then subjected to liquid chromatography, and the fractions containing the desired product were collected, concentrated and precipitated with an acid by adjusting the mixture to pH 2.0, to give a purified product. An equimolar amount of sodium bicarbonate was added to the above-purified produce and the mixture was freeze-dried to obtain a monosodium salt of the compound.

Melting Point, 257°C (decomposition).

IR (KBr):1760, 1660 $cm^{-1}$

$H^1$—NMR (90 MHz, $d_6$—DMSO):

$\delta$ ppm (pattern number of protons, assignment), 2.27 (s, 3H, $CH_3$), 3.45 (broad, 2H, 2—$CH_2$), 4.17 (broad, 2H, 3—$CH_2$), 4.77 (s, 2H, >N—$CH_2$—COO), 4.90 (d, 1H, 6—*CH*—), 5.63 (dd, 1H, 7—*CH*), 5.85

(d, 1H, —CONHC*H*—CO—),

6.21 (s, 1H, 5-proton on pyridine ring), 6.6, 7.15 (d, 4H, proton on phenyl ring), 8.17 (s, 1H, 2-proton on pyridine ring), 9.17 (d, 1H, —CON*H*—), 11.32 (d, —CON*H*—).

Example 2

The following compound was prepared according to the procedure as described in Example 1 with appropriate modification of the tetrazole reactant:

7-[D-α-(4-hydroxy-6-methylpyridine-3-carboxyamido)-α-(4-hydroxyphenyl)acetamido]-3-(1-sulfomethyl-1H-tetrazol-5-ylthiomethyl)-3-cephem-4-carboxylic acid monosodium salt.

Melting Point of 258°C (decomposition).

IR (KBr): 1750, 1655 ($cm^{-1}$)

$H^1$—NMR (90 MHz, $d_6$—DMSO):

δ ppm (pattern, number of protons, assignment), 2.33 (s, 3H, —$CH_3$), 3.0—3.8 (broad, 2—$CH_2$), 4.17 (broad, 2H, 3—$CH_2$—S—), 4.94—4.97 (m, 2H, 6—CH—, >N—$CH_2$—$SO_3$—), 5.70 (dd, 1H, 7—*CH*), 6.05 (d, 1H, —CONH—*CH*—CONH—), 6.27 (s, 1H, 5-proton on pyridine ring), 6.60 (d, 2H, proton on phenyl ring), 7.15 (d, 2H, proton on phenyl ring), 8.17 (s, 1H, 2-proton on pyridine ring), 9.20 (d, 1H, —CON*H*—), 11.53 (d, 1H, —CON*H*—).

Example 3

The following compound was prepared according to the procedure described in Example 1.

7-[D-α-(4-hydroxy-6-methylpyridine-3-carboxyamido)-α-(4 hydroxyphenyl)acetamido]-3-(1-carboxyethyl-1H-tetrazol-5-ylthiomethyl)-3-cephem-4-carboxylic acid.

Melting Point, 143—149°C (decomposition).

$H^1$—NMR ($d_6$—DMSO, 60 MHz):

δ ppm (pattern, number of protons, assignment), 2.27 (s, 3H, —$CH_3$), 2.93 (t, 2H, N—$CH_2CH_2COOH$), 3.62 (broad, 2H, 2—$CH_2$), 4.33 (m, >N—$CH_2CH_2COOH$), 4.97 (d, 1H, 6—*CH*), 5.67

(broad, 2H, —*CH*—, 7—*CH*),<br>
        |<br>
        NH

6.27 (s, 1H, 5-proton on pyridine ring), 6.83 (d, 2H, proton on phenyl ring), 7.27 (d, 2H, proton on phenyl ring), 8.27 (s, 1H, 2-proton on pyridine ring), 9.27 (d, 1H, —CON*H*—), 11.03 (d, 1H, —CON*H*—).

Example 4

The following compound was prepared according to the procedure described in Example 1:

7-[D-α-(4-hydroxy-6-methylpyridine-3-carboxyamido)-α-(4-hydroxyphenyl)acetamido]-3-(1-carboxypropyl-1H-tetrazol-5-ylthiomethyl)-3-cephem-4-carboxylic acid.

Melting Point, 182—183°C (decomposition).

$H^1$—NMR (60 MHz, $d_6$—DMSO):

δ ppm (pattern, number of protons, assignment), 1.8—2.4 (m, 7H, —$CH_3$, >N—$CH_2$—$CH_2$—), 3.63 (broad, 2H, 2—$CH_2$), 4.30 (broad, 4H, 3—$CH_2$, >N—$CH_2CH_2$—$CH_2COOH$), 4.98 (d, 1H, 6—*CH*—), 5.6—5.9

(m, 2H, —*CH*—, 7—*CH*),<br>
        |<br>
        NHCO—

6.28 (s, 1H, 5-proton on pyridine ring), 6.73 (d, 2H, proton on phenyl ring), 7.27 (d, 2H, proton on phenyl ring), 8.30 (s, 1H, 2-proton on pyridine ring), 9.30 (d, 1H, —CON*H*—), 11.1 (d. 1H, —CON*H*—).

Example 5

Preparation of 7-[D-α-(4-hydroxy-6-methylpyridine-3-carboxyamido)-α-(4-hydroxyphenyl)acetamido]-3-(1-pyridinio)methyl-1-cephem-4-carboxylate

A solution of 2.63 g of a triethylamine salt of 7-[D-α-(4-hydroxy-6-methylpyridine-3-carboxyamido)-α-(4-hydroxyphenyl)acetamido]cephalosporanic acid, 10 g of potassium thiocyanate and 0.481 g of pyridine in 10 ml of water was allowed to react at 58° to 60°C for 10 hours with stirring. The reaction mixture was allowed to cool to room temperature, 30 ml of water was added thereto and the insoluble substance formed was removed by filtration. The filtration was adjusted to pH 2 with 2N hydrochloric acid and, after stirring under ice cooling, the precipitated crystals were filtered and dried on phosphorus pentoxide under reduced pressure to obtain 1.60 g of a crude product of the named compound which was then purified by liquid chromatography.

$H^1$—NMR (60 MHz, $d_6$—DMSO):

δ ppm (pattern, number of protons, assignment), 2.27 (s, 3H, —$CH_3$), 3.43 (broad, 2H, 2—$CH_2$—), 5.07 (d, 1H, 6—*CH*—), 5.40—5.97

(m, —CH—, 7—*CH*—, 3—$CH_2$),
NH

6.27 (s, 1H, 5-proton on pyridine ring), 6.70. 7.23 (d, 4H, proton on phenyl ring), 8.18 (m, 2H, 3-proton of pyridinium), 8.27 (s, 1H, 2-proton on pyridine ring), 8.58 (d, 1H, 4-proton of pyridinium), 9.02 (d, 2H, 2-proton of pyridinium), 9.28 (broad d, 1H, —CON*H*—), 11.02 (broad d, 1H, —CON*H*).

Example 6

The following compound was prepared according to the procedure as described in Example 5: 7-[D-$\alpha$-(4-hydroxy-6-methylpyridine-3-carboxyamido)-$\alpha$-(4-hydroxyphenyl)acetamido]-3-(3-carboxy-1-pyridinio)-methyl-3-cephem-4-carboxylate.

$H^1$—NMR (60 MHz, $d_6$—DMSO):

$\delta$ ppm (pattern, number of protons, assignment), 2.25 (s, 3H, —$CH_3$), 3.42 (broad, 2H, 2—$CH_2$), 5.05 (d, 1H, 6—*CH*—), 5.3—6.0

(broad m, —*CH*—, 7-*CH*—, 3—$CH_2$),
NH

6.27 (s, 1H, 5-proton on pyridine ring), 6.70, 7.23 (m, 4H, proton on phenyl ring), 8.32 (m, 2H, 2-proton on pyridine ring, pyridinio proton), 8.67—9.67 (m, —CON*H*—, pyridinio proton), 11.07 (broad d, 1H, —CONH—).

Example 7

The following compound was prepared according to the procedure as described in Example 5: 7-[D-$\alpha$-(4-hydroxy-6-methylpyridine-3-carboxyamino)-$\alpha$-(4-hydroxyphenyl)acetamido]-3-(4-carbamoyl-1-pyridinio)-methyl-3-cephem-4-carboxylate.

$H^1$—NMR (60 MHz, $d_6$—DMSO):

$\delta$ ppm (pattern, number of protons, assignment), 2.27 (s, 3H, —$CH_3$), 3.45 (broad, 2H, 2—$CH_2$), 5.10 (d, 1H, 6-*CH*—), 5.27—6.13

(Broad m, 7—*CH*, —CH—3—$CH_2$),
NH

6.30 (s, 1H, 5-proton on pyridine ring), 6.72, 7.25 (m, 4H, proton on phenyl ring), 8.30 (s, 1H, 2-proton on pyridine ring), 8.50 (d, 3-proton of pyridinio), 11.08 (d, 1H, —CON*H*).

Example 8

The following compound was prepared according to the procedure as described in Example 5: 7-[D-$\alpha$-(4-hydroxy-6-methylpyridine-3-carboxyamido)-$\alpha$-(4-hydroxyphenyl)acetamido]-3-(4-carboxy-1-pyridinio)-methyl-3-cephem-4-carboxylate.

$H^1$—NMR (90 MHz, $d_6$—DMSO):

$\delta$ ppm (pattern, number of protons, assignment), 2.30 (s, 3H, —$CH_3$), 3,38 (broad, 2H, 2—$CH_2$), 5.03 (d, 1H, 6—*CH*), 5.2—6.0

(m, 4H, —*CH*—, 7-*CH*— 3—$CH_2$),
NH

6.27 (s, 1H, 5-proton on pyridine ring), 6.68, 7.22 (m, 4H, proton on phenyl ring), 8.33 (s, 1H, 2-proton on pyridine ring), 8.43 (d, 2H, 3-proton of pyridinio), 9.25 (d, 2H, 2-proton of pyridinio), 11.08 (d, 1H, —CON*H*—).

Preparation of 7-[D-$\alpha$-(4-hydroxylpyridine-3-carboxyamido)-$\alpha$-(4-hydroxyphenyl)acetamido]cephalosporanic acid

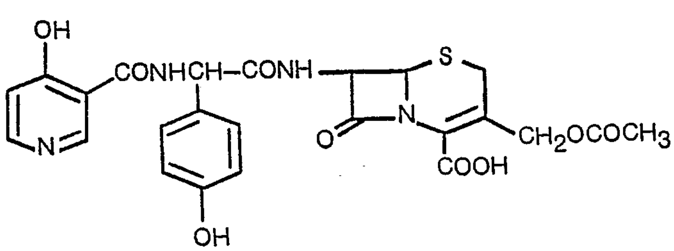

5 g of 7-[D-α-(*p*-methoxybenzyloxycarbonylamino)-α-(*p*-hydroxyphenyl)acetamido]cephalosporanic acid was added to a solution comprising 25 ml of trifluoroacetic acid and 2.5 ml of anisol under ice-cooling and stirring, and the mixture was stirred for 15 minutes. Then, the reaction mixture was added dropwise to 500 ml of diethyl ether while stirring. The precipitated crystals were collected by filtration, washed with diethyl ether, and dried over phosphorus pentoxide under reduced pressure to obtain 3.62 g of a trifluoroacetic acid salt of 7-[D-α-amino-α-(*p*-hydroxyphenyl)acetamido]cephalosporanic acid. Then, 3.45 g of 7-[D-α-amino-α-(*p*-hydroxyphenyl)acetamido]cephalosporanic acid and 1.52 g of 4-hydroxypyridine-3-carboxylic acid N-hydroxysuccinimide ester were added to 21 ml of dimethyl sulfoxide, and 1.95 g of triethylamine was added dropwise thereto at room temperature. After allowing the mixture to react for 40 minutes with stirring, 115 ml of acetone was added to the resulting reaction mixture which was then stirred for one hour. After stirring the mixture for 30 minutes under ice-cooling, the precipitated crystals were collected by filtration, washed with acetone and dried over phosphorus pentoxide under reduced pressure to afford 3.14 g of a triethylamine salt of the named compound. Melting Point, 155—157°C (decomposition).

Preparation of 7-[D-α-(4-hydroxy-6-methylpyridine-3-carboxyamido)-α-(4-hydroxyphenyl)-acetamido]-cephalosporanic acid

21.96 of anisol of 70.0 g of *p*-toluenesulfonic acid monohydrate were dissolved in 714 ml of acetonitrile, and 119 g of 7-[D-α-(*p*-methoxybenzyloxycarbonylamino)-α-(*p*-hydroxyphenyl)acetamido]-cephalosporanic acid was added thereto, followed by allowing the mixture to react for 40 minutes with stirring. Thereafter, 238 ml of dimethyl sulfoxide and then 49.2 g of 4-hydroxy-6-methylpyridine-3-carboxylic acid N-hydroxysuccinimide ester were added to the reaction mixture at 10°C, and 82.3 g of triethylamine was added dropwise thereto at that temperature.

After completion of the addition, the resulting mixture was stirred at the same temperature for 40 minutes and then further allowed to react at 15°C to 20°C for 1 hour with stirring. 2 l of acetone was added dropwise to the reaction mixture for 10 minutes and the mixture was stirred at 18°C for 30 minutes and then under ice-cooling for 1 hour. The crystals precipitated were collected by filtration, washed with 450 ml of acetone and dried over phosphorus pentoxide under reduced pressure to afford 95 g of a triethylamine salt of the named compound.
Melting Point, 160—173°C (decomposition).

**Claims**

1. A 3,7-substituted cephalosporin compound of the general formula:

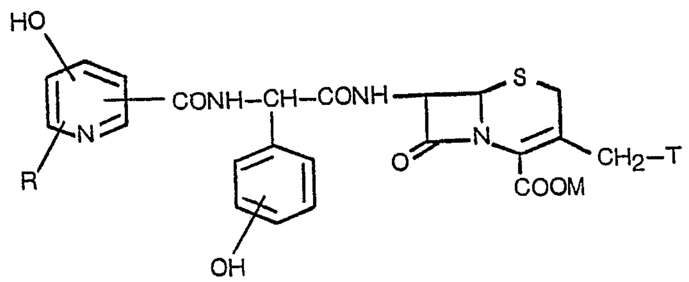

(I)

wherein R represents an alkyl group of up to 4 carbon atoms; T represents a group of one of the formulae:

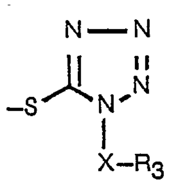

(a)

or

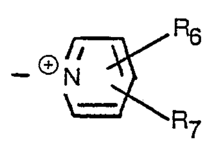

(b)

wherein $R_3$ represents a carboxyl or sulfo group, X represents an alkylene group of up to 4 carbon atoms, and $R_6$ and $R_7$ each represents a hydrogen atom, a hydroxy group, a carbamoyl group, a carboxy group or an alkyl group of up to 4 carbon atoms; M is a hydrogen atom, a pharmaceutically acceptable non-toxic cation, or when T is a group of the aforesaid formula (b), M may be an anionic charge.

2. A compound according to Claim 1, wherein the alkylene group represented by X is of the formula $-(CH_2)_n-$, wherein *n* is an integer of 1 to 4.

3. A compound according to Claim 1, wherein the group (b) is unsubstituted or $R^6$ or $R^7$ is a carboxyl or carbamoyl group.

4. A D-isomer of a compound as claimed in any of Claims 1 to 3.

5. A pharmaceutically acceptable non-toxic salt of a compound as claimed in any of Claims 1 to 4.

6. A process of producing a compound as claimed in any of Claims 1 to 5, which comprises reacting a compound of the general formula:

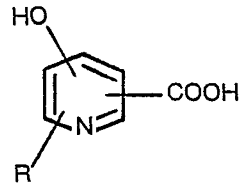

wherein R is as defined in Claim 1, or a reactive derivative thereof, with a compound of the general formula:

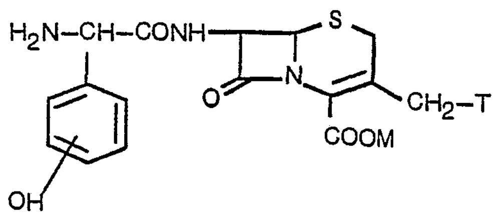

wherein T and M are as defined in Claim 1, or a reactive derivative thereof.

7. A process of producing a compound as claimed in any of Claims 1 to 5, which comprises reacting a compound of the general formula:

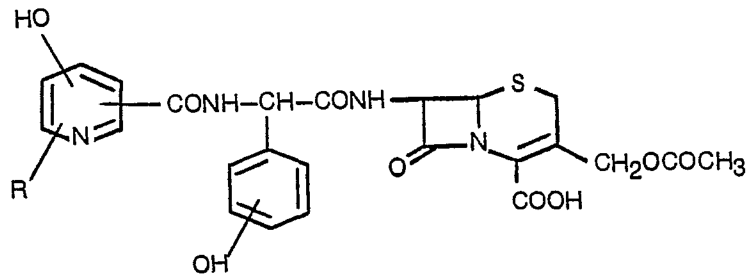

wherein R, $R_1$ and $R_2$ are as defined in Claim 1, or a salt thereof, with a compound of one of the formulae:

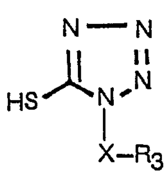

or

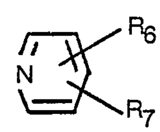

wherein $R_3$, $R_6$ and $R_7$ are as defined in Claim 1.

8. A process of producing a compound as claimed in any of Claims 1 to 5, which comprises reacting a compound of the general formula:

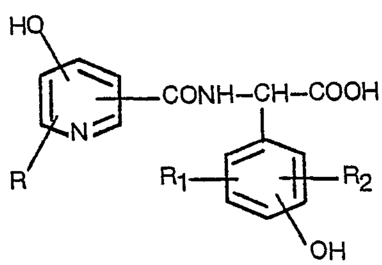

wherein R, $R_1$ and $R_2$ are as defined in Claim 1, or a reactive derivative thereof, with a compound of the formula:

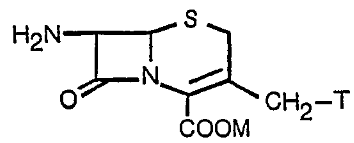

wherein T and M are as defined in Claim 1, or a salt or a derivative thereof.

9. An antimicrobial composition comprising an antimicrobially effective amount of a compound as claimed in any of Claims 1 to 4 or produced by a process as claimed in Claim 6, 7 or 8, and a carrier or diluent.

**Revendications**

1. Un dérivé de céphalosporine 3,7-substitué de formule générale:

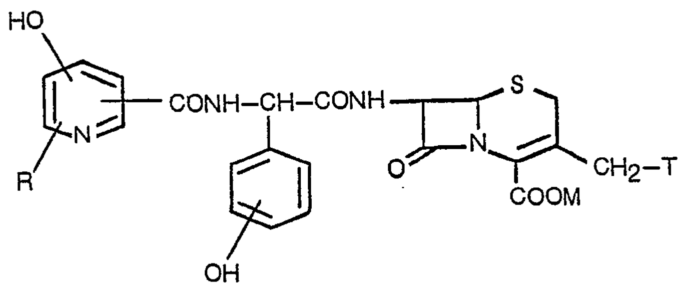

(I)

dans laquelle R représente un groupe alkyle ayant jusqu'à 4 atomes de carbone; T représente un groupe répondant à l'une des formules:

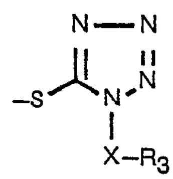 ou 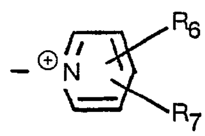

(a) (b)

dans lesquelles $R_3$ représente un groupe carboxy ou sulfo, X représente un groupe alkylène ayant jusqu'à 4 atomes de carbone et $R_6$ et $R_7$ représentent chacun un atome d'hydrogène, un groupe hydroxy, un groupe carbamoyle, un groupe carboxy ou un groupe alkyle ayant jusqu'à 4 atomes de carbone; M est un atome d'hydrogène, un cation non toxique acceptable en pharmacie ou, lorsque T est un groupe répondant à la formule (b) précédente, M peut être une charge anionique.

2. Un composé selon la revendication 1 dans lequel le groupe alkylène représenté par X a pour formule $-(CH_2)_n-$, où n est un entier de 1 à 4.

3. Un composé selon la revendication 1, où le groupe (b) est non substitué ou $R_6$ ou $R_7$ est un groupe carboxy ou carbamoyle.

4. Un isomère D d'un composé comme revendiqué dans l'une quelconque des revendications 1 à 3.

5. Un sel non toxique acceptable en pharmacie d'un composé comme revendiqué dans l'une quelconque des revendications 1 à 4.

6. Un procédé de production d'un composé comme revendiqué dans l'une quelconque des revendications 1 à 5, qui comprend la réaction d'un composé de formule générale:

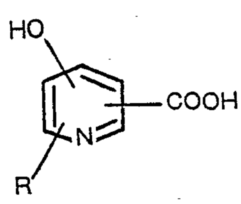

dans laquelle R est comme défini dans la revendication 1, ou d'un de ses dérivés réactifs, avec un composé de formule générale:

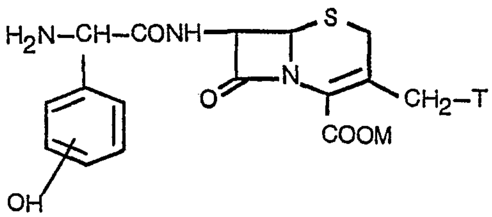

dans laquelle T et M sont comme défini dans la revendication 1 ou un de ses dérivés réactifs.

7. Un procédé de production d'un composé comme revendiqué dans l'une quelconque des revendications 1 à 5 qui comprend la réaction d'un composé de formule générale:

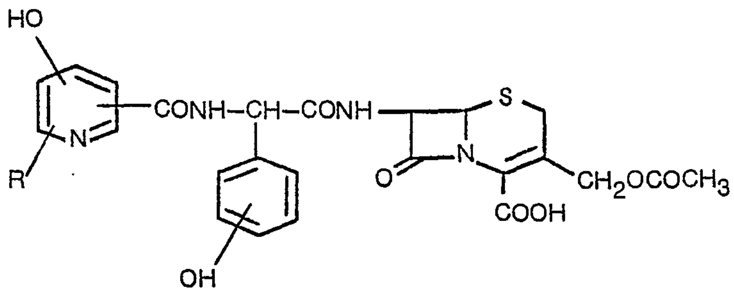

dans laquelle R, $R_1$ et $R_2$ sont comme défini dans la revendication 1 ou d'un de ses sels, avec un composé répondant à l'une des formules:

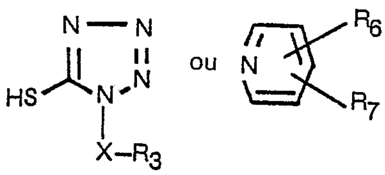

dans lesquelles $R_3$, $R_6$ et $R_7$ sont comme défini dans la revendication 1.

8. Un procédé de production d'un composé comme revendiqué dans l'une quelconque des revendications 1 à 5 qui comprend la réaction d'un composé de formule générale:

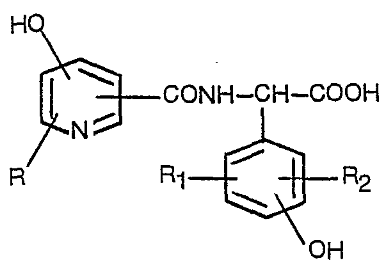

dans laquelle R, $R_1$ et $R_2$ sont comme défini dans la revendication 1 ou d'un de ses dérivés réactifs, avec un composé de formule:

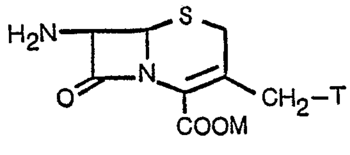

dans laquelle T et M sont comme défini dans la revendication 1, ou d'un de ses sels ou dérivés.

9. Une composition antimicrobienne comprenant une quantité antimicrobienne efficace d'un composé comme revendiqué dans l'une quelconque des revendications 1 à 4 ou produit selon un procédé comme revendiqué dans les revendications 6, 7 ou 8 et un support ou diluant.

**Patentansprüche**

1. Eine 3,7-substituierte Cephalosporinverbindung der allgemeinen Formel

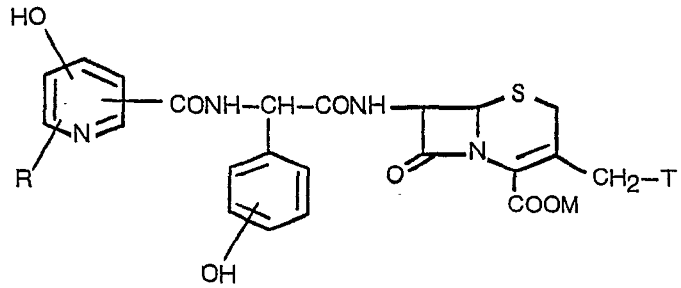

(I)

in der R einen Alkylrest mit bis zu 4 Kohlenstoffatomen bedeutet, T einen Rest einer der Formeln darstellt

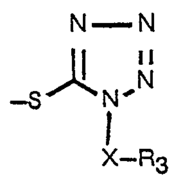 oder 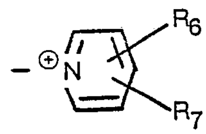

(a) (b)

wobei $R_3$ eine Carboxyl- oder Sulfogruppe bedeutet, X einen Alkylenrest mit bis zu 4 Kohlenstoffatomen darstellt und $R_6$ und $R_7$ jeweils ein Wasserstoffatom, eine Hydroxyl-, Carbamoyl-, Carboxy- oder Alkylgruppe mit bis zu 4 Kohlenstoffatomen bedeuten, M ein Wasserstoffatom oder ein pharmazeutisch verträgliches, nicht toxisches Kation ist, oder, wenn T einen Rest der vorstehenden Formel (b) darstellt, M eine anionische Ladung sein kann.

2. Verbindung nach Anspruch 1, wobei der durch X dargestellte Alkylenrest die Formel $-(CH_2)_n-$ hat, in der n eine ganze Zahl im Wert von 1 bis 4 ist.

3. Verbindung nach Anspruch 1, wobei der Rest (b) unsubstituiert ist oder $R_6$ oder $R_7$ eine Carboxyl- oder Carbamoylgruppe ist.

4. Ein D-Isomeres einer Verbindung nach einem der Ansprüche 1 bis 3.

5. Ein pharmazeutisch verträgliches, nicht-toxisches Salz einer Verbindung nach einem der Ansprüche 1 bis 4.

6. Ein Verfahren zur Herstellung einer Verbindung nach einem der Ansprüche 1 bis 5, das Umsetzung einer Verbindung der allgemeinen Formel

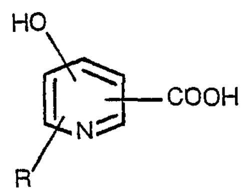

(II)

in der R wie in Anspruch 1 definiert ist, oder eines reaktionsfähigen Derivates davon, mit einer Verbindung der allgemeinen Formel

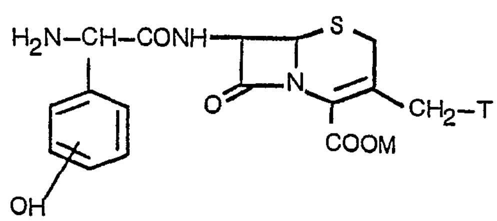

in der T und M wie in Anspruch 1 definiert sind, oder einem reaktionsfähigen Derivat davon, umfaßt.

7. Verfahren zur Herstellung einer Verbindung nach einem der Ansprüche 1 bis 5, das Umsetzung einer Verbindung der allgemeinen Formel

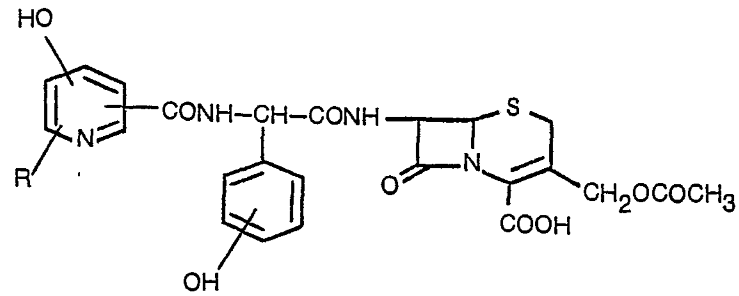

in der R, $R_1$ und $R_2$ wie in Anspruch 1 definiert sind, oder eines Salzes davon, mit einer Verbindung einer der Formeln

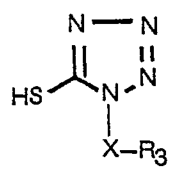

oder

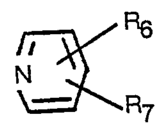

wobei $R_3$, $R_6$ und $R_7$ wie in Anspruch 1 definiert sind, umfaßt.

8. Ein Verfahren zur Herstellung einer Verbindung nach einem der Ansprüche 1 bis 5, das Umsetzung einer Verbindung der allgemeinen Formel

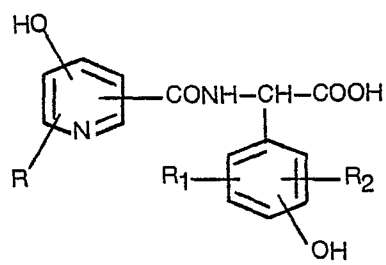

in der R, $R_1$ und $R_2$ wie in Anspruch 1 definiert sind, oder eines reaktionsfähigen Derivates davon, mit einer Verbindung der Formel

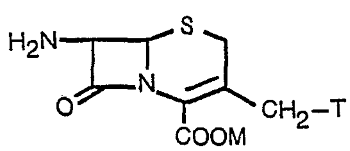

in der T und M wie in Anspruch 1 definiert sind, oder einem Salz oder einem Derivat davon, umfaßt.

9. Antimikrobielles Mittel, umfassend eine antimikrobiell wirksame Menge einer Verbindung nach einem der Ansprüche 1 bis 4 oder hergestellt nach einem Verfahren gemäß Anspruch 6, 7 oder 8, und einen Träger oder ein Verdünnungsmittel.